# EUROPEAN PATENT SPECIFICATION

(11) **EP 3 829 547 B1**
(45) Date of publication and mention of the grant of the patent: **03.09.2025**
(21) Application number: 19749663.1
(22) Date of filing: 26.07.2019
(51) Int. Cl.: A61K 9/20, A61K 31/519

(54) **PHARMACEUTICAL COMPOSITION OF TICAGRELOR**
PHARMAZEUTISCHE ZUSAMMENSETZUNG VON TICAGRELOR
COMPOSITION PHARMACEUTIQUE DE TICAGRÉLOR

(30) Priority: 27.07.2018 SI 201800167
(43) Date of publication of application: 09.06.2021
(73) Proprietor: KRKA, d.d., Novo mesto, 8000 Novo mesto (SI)
(72) Inventor: BEZNEC, Tea, 9252 Radenci (SI); KORASA, Klemen, 8501 Novo mesto (SI); BASELJ, Nastja, 8501 Novo mesto (SI); HOCEVAR, Tina, 1211 Ljubljana Smartno (SI); CERNOSA, Lidia, 8311 Kostanjevica na Krki (SI); KOLENC, Ivanka, 8000 Novo mesto (SI)
(74) Representative: Uexküll & Stolberg
(86) International application number: PCT/EP2019/070273
(87) International publication number: WO 2020/021110

(56) References cited:
- WO-A1-2017/182455
- WO-A2-2011/076749
- ANONYMOUS: "Ethanol", TECHNICAL EVALUATION REPORT COMPILED BY PESTICIDE RESEARCH INSTITUTE FOR THE USDA NATIONAL ORGANIC PROGRAM, 2 January 2014 (2014-01-02), pages 1 - 21, XP055628736, Retrieved from the Internet <URL:https://www.ams.usda.gov/sites/default/files/media/Ethanol%201%20TR%202014.pdf> [retrieved on 20191003]
- "The United States Pharmacopeia USP 24. The National Formulary NF 19", 1999, UNITED STATES PHARMACOPEIAL CONVENTION INC, Rockville, ISBN: 978-1-88-978803-6, article "Solubility", pages: 10, XP055026035

## Description

### Field of the invention

The present invention relates to a pharmaceutical composition which comprises ticagrelor and is free of water soluble fillers/diluents/carriers. The pharmaceutical composition according to the invention is cost effective and enables the required processability, dissolution profile and bioavailability of the drug.

### Background of the invention

Ticagrelor is a common name for (1S,2S,3R,5S)-3-[7-{[(1R,2S)-2-(3,4-difluorophenyl) cyclopropyl]amino}-5-(propylthio)-3H-[1,2,3]triazolo[4,5-d]pyrimidin-3-yl]-5-(2-hydroxyethoxy) cyclopentane-1,2-diol, a compound of formula (I):

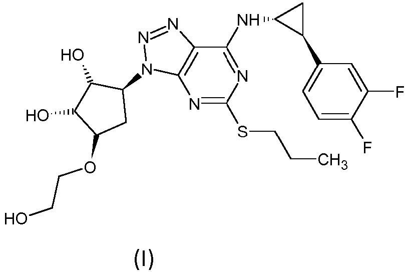

Ticagrelor is a BCS class IV compound since it has a low soluble active substance (not ionised in the physiological pH range) and exhibits a moderate intrinsic permeability. Therefore, there is potentially a higher risk that changes in formulation and processing parameters can affect clinical performance. Moreover, this low aqueous solubility of ticagrelor leads to an increase of relevance of particle size.

Ticagrelor is available on the market and is sold under trade name Brilique, as film coated tablets in 90 mg and 60 mg strengths. The tablet comprises mannitol (as a diluent), dibasic calcium phosphate (as a diluent), sodium starch glycolate (as a disintegrant), hydroxypropyl cellulose (as a binder), and magnesium stearate (as a lubricant). In the process of production of Brilique purified water is used as a granulation fluid.

Ticagrelor is a reversible, selective P2Y12-receptor antagonist (anti-platelet agent) being developed to reduce thromboembolic events in patients with atherosclerosis. It is orally active and does not require metabolic activation. Ticagrelor, co-administered with acetylsalicylic acid (ASA), is indicated for the prevention of atherothrombotic events in adult patients with acute coronary syndromes (ACS) or a history of myocardial infarction (MI) and a high risk of developing an atherothrombotic event.

EP 1135391 B1 discloses triazolo[4,5-d]pyrimidine compounds like ticagrelor, processes for their production, their use and pharmaceutical compositions thereof. The patent does not disclose any specific pharmaceutical composition of ticagrelor.

WO 2001/092262 relates to specific crystalline forms of ticagrelor selected from forms I, II, III, IV, and amorphous form α. The polymorphic form which is present in the marketed formulation is form II. The document *inter alia discloses* the composition of a tablet comprising the active ingredient in admixture with an adjuvant or a carrier such as lactose, saccharose, sorbitol, mannitol, starches, cellulose derivatives, a binder such as gelatine or polyvinylpyrrolidone, and a lubricant. Specific examples of tablets are disclosed all using lactose as a diluent.

WO 2011/076749 discloses a pharmaceutical dosage form of ticagrelor wherein at least 90% by volume of ticagrelor particles have a particle size in the range from 1 µm to 150 µm. The document discloses pharmaceutical compositions comprising lactose, mannitol or isomalt as a diluent.

In WO 2008/024045 and WO 2008/024044 a pharmaceutical composition of ticagrelor is disclosed which comprises a filler that is a combination of mannitol and dibasic calcium phosphate dihydrate, a binder selected from sodium starch glycolate and one or more lubricants. Hence, the document teaches a combination of soluble and insoluble fillers.

In the Declaration of K. Purdy, Ph.D., submitted for US Serial No. 11/841,030, obtainable via USPTO database Public Pair, three different formulations of ticagrelor are disclosed. In the first formulation a mixture of lactose and microcrystalline cellulose was used as a diluent.

The manufacturing process resulted in low yields, variable granule size distribution and poor tablet compression properties so that this formulation was not suitable for large scale production. In the second formulation lactose and microcrystalline cellulose were replaced by mannitol and dibasic calcium phosphate dihydrate. This formulation resulted in improved formulation in terms of improved processing, dissolution and bioavailability. The third formulation was further improved in terms of extension of shelf life stability by replacing the binder/disintegrant combination povidone/croscarmellose sodium with hydroxypropyl cellulose/sodium starch glycolate. This document shows that any changes in the formulation of ticagrelor significantly influence the clinical performance, processability and stability.

EP 2908802 discloses a composition of ticagrelor comprising non-hygroscopic filler and non-hygroscopic binder. As a non-hygroscopic filler glucose, fructose, sucrose, lactose monohydrate, anhydrous lactose, raffinose, isomaltose, trehalose, dextrates, mannitol, erythritol, sorbitol, maltitol, xylitol and lactitol, compressible sugars, calcium hydrogen phosphate dihydrate and their mixtures are mentioned. Particularly, water-soluble fillers such as lactose, mannitol and xylitol are preferred.

WO 2015/110952 discloses pharmaceutical compositions which are free of water insoluble fillers. Specifically mentioned water soluble fillers are dextrose, fructose, lactose, maltitol, maltodextrins, maltose, sorbitol, sucrose, xylitol, erythritol and the like.

WO 2013/150495, WO 2014/118808, WO 2014/059955, WO 2014/170026, WO 2015/001489, WO 2015/039513 and WO 2017/182455 disclose pharmaceutical compositions comprising amorphous ticagrelor or solid dispersions containing amorphous ticagrelor. It is known that the amorphous form generates more impurities if compared with the crystalline forms, making the use of amorphous form in a commercial scale very difficult. Additionally, the amorphous form of ticagrelor poses more challenges during formulation preparation due to its physico-chemical properties. It is reported that using amorphous form of ticagrelor in formulation causes problems when amorphous ticagrelor comes in contact with moisture because it forms a cohesive mass which prolongs the disintegration time as well as retards the dissolution of a formulation. Further, it is very difficult and cumbersome to prepare a formulation which contains a cohesive mass and to characterize it. Moreover, preparation of solid dispersions demands use of special equipment, it is time consuming and is not favourable from economical point of view.

EP 3332769 discloses a solid pharmaceutical composition comprising ticagrelor with at least one hydrophilic polymer wherein the composition is substantially free of calcium salts and derivates thereof. In the exemplified formulation a mixture of microcrystalline cellulose and mannitol was used as a filler combination.

As evident from the prior art there are several pharmaceutical compositions comprising ticagrelor dealing with a problem of formulating ticagrelor. The disclosed compositions comprise inactive ingredients comprising water soluble fillers; in most cases mannitol is used. From the economical point of view mannitol is not the favourable option for filler selection due to its price. Moreover, water soluble fillers such as lactose and fructose may cause problems in patients which suffer from lactose or fructose intolerance, whereas sugar alcohols such as mannitol or sorbitol may have laxative effect.

Thus, there exists an enduring need to provide an improved formulation of ticagrelor which solves the above problems and in particular provides for uniform and complete release of the drug. Moreover, it would be desirable to provide a formulation of ticagrelor which is suitable for use on a commercial scale and is cost efficient in terms of excipients used and also the manufacturing process used.

### Summary of the invention

The above problems are solved by a pharmaceutical formulation comprising (a) ticagrelor and (b) at least two water insoluble fillers, wherein the formulation is free of water soluble fillers. The filler (b) is a mixture of at least two water insoluble fillers, such as a mixture of a polysaccharide, most preferably microcrystalline cellulose, and a calcium, sodium or potassium salt of phosphoric acid, carbonic acid or sulphuric acid, most preferably calcium hydrogen phosphate. Hence, in a preferred embodiment the present invention is directed to a pharmaceutical formulation comprising (a) ticegrelor and (b) a mixture of two fillers selected from the group consisting of polysaccharides and calcium, sodium or potassium salts of phosphoric acid, carbonic acid or sulphuric acid, most preferably a mixture of microcrystalline cellulose and calcium hydrogen phosphate.

According to the present invention there is disclosed a pharmaceutical formulation comprising (a), as the active ingredient, ticagrelor, (b) at least two water insoluble fillers, (c) at least one binder, (d) at least one disintegrant and (e) optionally further pharmaceutically acceptable excipients, wherein the formulation is free of water soluble fillers.

According to a preferred embodiment of the present invention there is disclosed a pharmaceutical formulation comprising (a), as the active ingredient, ticagrelor, (b) a mixture of at least two water insoluble fillers, (c) at least one binder, (d) at least one disintegrant and (e) optionally further pharmaceutically acceptable excipients, wherein the formulation is free of water soluble fillers.

According to the preferred embodiment of the present invention there is disclosed a pharmaceutical formulation comprising (a), as the active ingredient, ticagrelor, (b) a mixture of at least two water insoluble fillers selected from polysaccharides, inorganic calcium salts and derivates thereof, (c) at least one binder selected from hydrophilic cellulose derivatives, (d) at least one disintegrant and (e) optionally further pharmaceutically acceptable excipients, wherein the formulation is free of water soluble fillers.

The present invention is also directed to a wet granulation process for the preparation of a pharmaceutical formulation according to the present invention, which comprises:
- blending ticagrelor and at least one excipient or a mixture of excipients, which excipient(s) is/are optionally sieved, to give a granulation mixture;
- granulating the granulation mixture using granulating liquid which comprises water to give a granulate;
- optionally sieving the granulate;
- adding lubricant to the granulate to give a compression mixture;
- compressing the compression mixture to the desired solid dosage form or encapsulating the compression mixture;
- optionally, applying a film coating.

### Figures

Figure 1 shows dissolution profiles of Reference Example and Examples 1 to 5.
Figure 2 shows dissolution profiles of Example 1 and Example 6.
Figure 3 shows dissolution profiles of Example 2 and Example 7.

### Detailed description of the invention

### Definitions

The term **"water soluble filler"** means an excipient, which is substantially soluble in water at room temperature. As used herein, the term "water soluble filler" means that dissolving of 1g of filler requires less than 30mL of water at a temperature of 25°C, more preferably less than 10 mL of water, most preferably less than 6 mL of water.

The term **"water insoluble filler"** means an excipient which has low or slow solubility in water at room temperature. As used herein, the term "water insoluble" means that dissolving of 1g of filler requires more than 30mL of water at a temperature of 25°C, preferably more than 100 mL of water, most preferably more than 1000 mL of water. Solubility of the filler is determined in terms of the number of parts by volume of water required to dissolve one part by weight of the filler. As know by those skilled in the art, solubility is determined once the state of equilibrium is reached. In particular, the solubility characteristics are presently determined with a shaking flask method perfomed at 25°C for 24 hours.

The terms **filler/diluent/carrier** can be used interchangeably and mean fillers in pharmaceutical tablets used to increase weight and improve content uniformity. Fillers are used to increase the bulk volume of a tablet and to dilute active pharmaceutical ingredients to obtain adequate weight and size of a pharmaceutical composition.

The term **excipient** has its normal meaning, i.e. it refers to any pharmaceutically acceptable substance that has no therapeutic activity as such but is present in the formulation for other reasons, e.g. to improve dissolution of the active pharmaceutical ingredient (API) itself. Pharmaceutically acceptable excipients may for instance be selected from diluents/fillers, lubricants and/or glidants, disintegrants and binders.

The term **"tablet"** as used herein is intended to encompass compressed pharmaceutical dosage formulations of all shapes and sizes. The term "capsule" encompasses pharmaceutical dosage form formulations wherein the active pharmaceutical ingredient, optionally mixed with further excipients, is enclosed in a capsule shell.

The terms **"formulation"** and **"composition"** can be used interchangeably to denote a composition for pharmaceutical use or a pharmaceutical dosage form formulation.

The term **average particle size** refers to the volume average or volume mean diameter of the particle size distribution. The average particle size can be determined by laser light scattering using e.g Malvern Mastersizer equipped with wet dispersion unit. Particle sizes are determined by measuring the angular distribution of laser light scattered by a homogeneous suspension of particles. The particles to be subjected to the particle size measurement are first suspended in appropriate dispersant such as vegetable oil and then subjected to a size determination in a Malvern Mastersizer instrument. Usually, 100-800 mg of substance is dispersed in 5-10 mL of dispersant. Values d(0.1), d(0.5) and d(0.9) indicate that 10%, 50% and 90% of the particles, respectively, are smaller than the specified values.

Unless specified otherwise, all percentages indicated herein are % by weight based on the total weight of the pharmaceutical formulation.

### Overview

During their work the present inventors have observed that compositions comprising ticagrelor that meet the requirements for a processible, stable and bioequivalent formulation can be prepared using water insoluble fillers only, which is contrast to the teaching of the prior where presence of water soluble filler was obligatory for preparing a product with desired properties.

It has been found out that the desired storage stability, *in-vitro* dissolution and processability of pharmaceutical compositions of ticagrelor are highly dependent on the nature of the inactive pharmaceutical ingredients used.

Most of the pharmaceutical compositions disclosed in the prior art comprise lactose and sugar alcohols, such as mannitol. Lactose is known for side effects in patients with lactose intolerance, therefore its use should be avoided in pharmaceutical composition. Furthermore, sugar alcohols such as mannitol, sorbitol are known to have a laxative effect. Therefore, it was the aim of inventors to develop a composition wherein excipients having potential adverse effects are replaced by excipients having less adverse effect and being at the same time cost efficient.

It has been unexpectedly found out that target processability, storage stability and dissolution profile similar to that obtained for the marketed product Brilique can be achieved by a composition which comprises water insoluble fillers and is free of water soluble fillers. Surprisingly, a target dissolution profile can be observed even if active ingredient having a different particle size is used.

The present invention provides pharmaceutical formulations that allow accomplishing excellent process characteristics while maintaining excellent dissolution characteristics of ticagrelor and suitability for industrial scale production.

As shown by the Comparative Example 1, the pharmaceutical composition of the present invention provides for a more robust technological process which is less susceptible for variation in the ratio of granulation liquid to granulation mass prior to granulation. In case water soluble filer is used less variations in amount of granulation liquid are allowable, whereas the composition of the present inventions allows broader ranges in the amount of granulation liquid used.

### The pharmaceutical formulation

The following characteristics are common to the pharmaceutical formulation of the various embodiments of the invention.

The pharmaceutical formulations of the present invention are solid pharmaceutical formulations. The formulations can be in the form of capsules, powders, tablets, minitablets, microtablets, coated tablets, coated minitablets, coated microtablets, pills, lozenges, etc. The pharmaceutical formulations of the present invention are preferably suitable for oral application. The formulations of the present invention preferably have the form of capsules or tablets.

The pharmaceutical compositions of the present invention are preferably formulated in a unit dosage form, each dosage form containing about 30 to about 180 mg of ticagrelor, preferably oral dosage form comprising 60 mg or 90 mg of ticagrelor.

### The active pharmaceutical ingredient

The following characteristics of the API apply to all embodiments of the present invention.

The component (a), ticagrelor, can be selected from any crystalline form such as disclosed in e.g. WO 2001/092262, EP 2816043, WO 2015/037016, WO 2017/118633.

According to one aspect of any one of the embodiments of the present invention, the ticagrelor has an average particle size in the range of 1 and 30 µm, preferably between 2 and 20 µm and most preferably between 3 and 10 µm.

In another aspect of any of the embodiments of the present invention, ticagrelor has a particle size distribution of d(0.1) from 0.1 to 20 µm, d(0.5) from 1 to 30 µm, and d(0.9) from 3 to 100 µm, such as d(0.1) from 0.1 to 20 µm, d(0.5) from 1 to 30 µm, and d(0.9) from 5 to 100 µm, preferably of d(0.1) from 0.5 to 10 µm, d(0.5) from 2 to 20 µm, and d(0.9) from 7 to 100 µm, such as d(0.1) from 0.5 to 10 µm, d(0.5) from 2 to 20 µm, and d(0.9) from 5 to 100 µm.

According to a preferred embodiment, the present invention is directed to a pharmaceutical formulation comprising ticagrelor in a crystalline form, and more preferably crystalline ticagrelor having an average particle size as specified above.

### Excipients

The **water insoluble filler/diluent/carrier** (a) can be selected from the group consisting of water insoluble polysaccharides, salts of alkaline or earth alkaline metals and mixtures thereof, and preferably is selected from cellulose and calcium, sodium or potassium salts of phosphoric acid, carbonic acid or sulfuric acid and mixtures thereof. The polysaccharide is preferably a polysaccharide comprising from 200 to 10,000 monosaccharide residues, preferably 500 to 10,000 monosaccharide residues, preferably glucose residues. In particular, the polysaccharide is cellulose. Cellulose may be selected from powdered cellulose, microcrystalline cellulose and silicified microcrystalline cellulose, and most preferably microcrystalline cellulose is used. Calcium salts of phosphoric acid, carbonic acid or sulfuric acid may be preferably selected from calcium hydrogen phosphate anhydrous or hydrate, calcium phosphate, calcium sulphate and calcium carbonate, and most preferably is calcium hydrogen phosphate.

The pharmaceutical formulation according to the invention is free of water soluble fillers or diluents. The **water soluble filler or diluent** can be selected from the group consisting of monosaccharides, oligosaccharides, sugar alcohols and water soluble polysaccharides, such as starch. Starch may be selected from starches from any suitable botanical source, for example corn, wheat, rice, tapioca and potato. Preferably, starches are modified starches, such as pregleatinized starch, which is a type of modified starch that has been processed to render the starch more flowable and directly compressible. Partially or wholly pregelatinized starches can be used. Monosaccharides, oligosaccharides and sugar alcohols may be selected from glucose, fructose, sucrose, lactose monohydrate, anhydrous lactose, raffinose, isomaltose, trehalose, dextrates, mannitol, erythritol, sorbitol, maltitol, xylitol, lactitol, compressible sugars and mixtures thereof. Preferably, the filler or diluent of the formulation according to the invention is not selected from water soluble diluent/filler.

According to a particularly preferred embodiment, the filler or diluent (a) of the formulation according to the invention is selected from cellulose powders (Ph. Eur.), microcrystalline celluloses and/or calcium salt of phosphoric acid. More preferably, the pharmaceutical formulation of the present invention comprises at least one filler or diluent selected from microcrystalline cellulose or calcium hydrogen phosphate or a mixture thereof.

Microcrystalline cellulose is preferably selected from microcrystalline cellulose with an average particle size from 10 µm to 200 µm, preferably from 20 to 150 µm and/or moisture content ≤ 5%. Particularly, microcrystalline cellulose may be selected from microcrystalline cellulose with an average particle size of 20 µm and a moisture content ≤ 5%, such as Avicel PH-105, microcrystalline cellulose with an average particle size of 50 µm and a moisture content ≤ 5%, such as Avicel PH-101 or Vivapur 101, microcrystalline cellulose with an average particle size of 50 µm and a moisture content ≤ 2%, such as Avicel PH-113, microcrystalline cellulose with an average particle size of 90 to 120 µm and a moisture content ≤ 5%, such as Avicel PH-102 or Vivapur 102, microcrystalline cellulose with an average particle size of 90 to 120 µm and a moisture content ≤ 1.5%, such as Avicel PH-112.

It has been surprisingly found that the use of a mixture of microcrystalline cellulose and calcium hydrogen phosphate does not only result in formulations with desired dissolution properties, but also allows to prepare formulations with superior procesability, weight variation and flow properties.

The total amount of filler or fillers, in case a combination of fillers is used, in the formulation according to the invention is typically in the range of about 30% to about 90% by weight. Preferably, the amount of the filler is about 40% to about 80%, more preferably about 50% to about 70%, by weight of the formulation.

The pharmaceutical formulations of the present invention may contain additional ingredients selected from a wide variety of excipients known in the art of pharmaceutical formulation, such as disintegrants, binders, lubricants, and glidants.

Suitable **disintegrants** (d) may be selected from crospovidone, starch, maize starch, pregelatinized starch, sodium starch glycollate, hydroxypropyl starch, microcrystalline cellulose, sodium and/or calcium salts of carboxymethyl cellulose, cross-linked carboxymethylcellulose (e.g. croscarmellose sodium and/or croscarmellose calcium), polacrilin potassium, low substituted hydroxypropylcellulose (L-HPC), sodium and/or calcium alginate, docusate sodium, methylcellulose, agar, guar gum, chitosan, alginic acid or mixtures thereof. According to one aspect of the invention, it is preferred to use a disintegrant selected from sodium and/or calcium salts of carboxymethyl cellulose, cross-linked carboxymethylcellulose (e.g. croscarmellose sodium and/or croscarmellose calcium). Preferably, disintegrants are selected from cross-linked carboxymethylcellulose (e.g. croscarmellose sodium and/or croscarmellose calcium).

Suitable **binders** (c) may be selected from povidone (polyvinylpyrrolidone), copovidone (vinylpyrrolidone-vinyl acetate copolymer), cellulose derivatives such as hydroxymethylcellulose, hydroxyethylcellulose, hydroxypropylcellulose, hydroxypropylmethylcellulose, low viscosity hydroxypropylmethylcellulose and/or other cellulose ethers. Preferably, low viscosity hydroxypropylmethylcellulose is used.

**Lubricants and glidants** (e) are preferably used in the formulations of the invention in the usual standard way.

Suitable lubricants may be selected from fatty acids, fatty acid esters, including glyceride esters (e.g., selected from glyceryl monostearate, glyceryl tribehenate, and glyceryl dibehenate), the group of metal salts of fatty acids with 12 to 20 carbon atoms such as magnesium, calcium, aluminum or zinc stearate, magnesium palmitate and magnesium oleate, hydrogenated vegetable oil, hydrogenated castor oil, talc, wax (e.g. STEROTEX NF, Lubriwax (Hydrogenated Vegetable Oil Type), meads wax or spermaceti), boric acid, sodium stearyl fumarate, macrogols, sugar esters (e.g., sorbitan monostearate and sucrose monopalmitate), inorganic materials (e.g., talc), polymers (e.g., PEG) or mixtures thereof. Preferably, lubricants are selected from magnesium stearate, sodium stearyl fumarate or stearic acid.

The pharmaceutical formulation of the present invention can furthermore comprise one or more **glidants.** In some aspects, the one or more glidants are selected from colloidal silica, talc and magnesium trisilicate.

In preferred aspects of the invention, the active ingredient is evenly distributed in a matrix formed by the other ingredients of the formulation. According to a more preferred embodiment, the pharmaceutical formulation of the present invention comprises:

| | |
|---|---|
| ticagrelor: | 5-80%, more preferably 20-50%, most preferably 25-40% by weight; |
| filler/diluent: | 30-90 %, more preferably 40-80 %, most preferably 50-70 % by weight; |
| binder: | 0-15 %, more preferably 1.5-10 %, most preferably 2-5 % by weight; |
| disintegrant: | 0-15 %, more preferably 1.5-10 %, most preferably 2-5 % by weight; |
| lubricant: | 0.25-5 %, more preferably 0.5-3 %, most preferably 0.5-2 % by weight; |
| glidant: | 0-10 %, more preferably 0-5 %, most preferably 0-3 % by weight; |

wherein the pharmaceutical formulation is free of water soluble diluents/fillers.

In a preferred aspect, the formulation of the invention comprises 20% to 50% by weight of ticagrelor, 40 to 80 % by weight of a filler/diluent selected from microcrystalline cellulose and calcium hydrogen phosphate, 1.5 to 10 % by weight of a binder selected from low viscosity hydroxypropylmethylcellulose and 1.5 to 10 % by weight of an disintegrant preferably selected from croscarmellose sodium and 0. 5 to 3 % by weight of a lubricant, preferably selected from magnesium stearate.

In another preferred aspect, the formulation of the invention comprises (a) 5 to 80%, more preferably 20 to 50%, most preferably 25 to 40% by weight, of ticagrelor, and in particular of crystalline ticagrelor, and (b) 30 to 90%, more preferably 40 to 80 %, most preferably 50 to 70 % by weight of the water insoluble filler.

In another preferred aspect, the formulation of the invention comprises (a) 5 to 80%, more preferably 20 to 50%, most preferably 25 to 40% by weight, of ticagrelor, and in particular of crystalline ticagrelor, (b) 30 to 90%, more preferably 40 to 80 %, most preferably 50 to 70 % by weight of the water insoluble filler, and (c) 0 to 15 %, more preferably 1.5 to 10 %, most preferably 2 to 5 % by weight of the binder.

In another aspect, the present invention is directed to a pharmaceutical formulation comprising components (a) to (e), as described above, and preferably in the amounts as described above, and comprising less than 5 % by weight, preferably less than 2 % by weight and more preferably less than 1 % by weight of water soluble fillers or diluents, as described above. Most preferably, such a pharmaceutical formulation is substantially free or free of water soluble fillers or diluents.

### Method of the present Invention

The pharmaceutical formulations of the present invention may be manufactured using wet or dry methods. The present invention provides, as a further embodiment, methods for preparing the formulations of the present invention.

According to a preferred aspect, the pharmaceutical formulations are prepared by wet methods such as wet granulation.

The present inventors have found out that the formulations prepared by wet processes exhibit an improved dissolution profile compared to the formulations prepared by dry processes.

The methods described below are intended for preparing the pharmaceutical formulations of the present invention. This means that the ingredients (API, excipients) mentioned below must satisfy the essential features described for the pharmaceutical formulations of the present invention and that they preferably satisfy the features described as preferred features of the pharmaceutical formulations of the present invention.

The wet granulation process according to the present invention comprises a process which comprises:
- blending optionally sieved ticagrelor with at least one excipient or a mixture of excipients to obtain a granulation mixture;
- granulating the granulation mixture with a granulation liquid using processes known in the art, such as fluid bed granulator or high shear granulator, to obtain a granulate;
- optionally drying, milling and sieving the obtained granulate to obtain a granulate with the desired particle size distribution;
- adding the rest of the at least one excipient or a mixture of excipients to the granulate to give a mixture; and
- compressing the mixture to the desired solid dosage form or encapsulating the mixture.

Granulation liquids can be selected from water, organic solvents or mixtures thereof. Organic solvents used in granulation for the preparation of the composition of the present invention are selected from alcohols with 1 to 4 carbon atoms like absolute ethanol, concentrated alcohol (96 vol%), methanol, isopropropanol, ketons such as acetone or esters such as ethylacetate or mixtures thereof. Preferably, water is used as a granulation liquid.

Excipients selected from diluents, binders, disintegrants, lubricants and glidants can optionally be dissolved, suspended, emulsified or dispersed into the granulation liquid which is sprayed onto the powder mixture comprising ticagrelor and at least one excipient or a mixture of excipients. Preferably, binders are dissolved in the granulation liquid. Optionally, the complete amount of binder present in the composition is dissolved in the granulation liquid or, alternatively, only a portion of a binder present in the composition, such as ¼ or ½ of the complete amount of binder, is dissolved in the granulation liquid.

Preferably, a wet granulation process for preparation of the pharmaceutical formulation according to the present invention comprises the following steps:
- blending ticagrelor and optionally sieved at least one excipient or a mixture of excipients to give a granulation mixture;
- granulating the granulation mixture using a granulation liquid which comprises water and optionally a binder to give granulate;
- optionally drying, milling and sieving the obtained granulate;
- adding lubricant to the granulate to give a compression mixture;
- compressing the compression mixture to the desired solid dosage form or encapsulating the compression mixture; and
- optionally, applying a film coating.

The following examples illustrate the invention and are not intended to restrict the scope of the invention, as defined by the appended claims, in any way.

### Examples

### Dissolution

Dissolution profile was determined in a system comprising purified water with 0.2 % of Polysorbate 80, in 900 mL using USP Apparatus II (Paddle) method at 75 rpm.

In the Examples ticagrelor in polymorphic form II as disclosed in WO 2001/092262 was used.

### Examples 1-5

**Examples according to the invention and reference example for preparing pharmaceutical compositions are disclosed in Table 1 below:**

**Table 1**

| | **Ref. Example** | Example 1 | Example 2 | Example 3 | Example 4 | Example 5 |
|---|---|---|---|---|---|---|
| | [mg/tablet] | | | | | |
| Ticagrelor (particle size approx. 3 µm) | 90 | 90 | / | / | / | / |
| Ticagrelor (particle size approx. 6 µm) | / | / | 90 | / | / | / |
| Ticagrelor (particle size approx. 9 µm) | / | / | / | 90 | / | / |
| Ticagrelor (particle size approx. 13 µm) | / | / | / | / | 90 | / |
| Ticagrelor (particle size approx. 35 µm) | / | / | / | / | / | 90 |
| Microcrystalline cellulose, type 101 | / | 126 | 126 | 126 | 126 | 126 |
| Calcium hydrogen phosphate | 63 | 63 | 63 | 63 | 63 | 63 |
| Hydroxypropyl methylcellulose 6CP/PHARMACOAT 606 | / | 9 | 9 | 9 | 9 | 9 |
| Hydroxypropylcellulose (Klucel EF) | 9 | / | / | / | / | / |
| Mannitol | 126 | / | / | / | / | / |
| Sodium croscarmellose | / | 9 | 9 | 9 | 9 | 9 |
| Sodium starch glycolate | 9 | / | / | / | / | / |
| Magnesium stearate | 3 | 3 | 3 | 3 | 3 | 3 |

| Coating | | | | | | |
|---|---|---|---|---|---|---|
| Coating mixture | 8.9 | 8.9 | 8.9 | 8.9 | 8.9 | 8.9 |
| Ferrous oxide yellow | 0.1 | 0.1 | 0.1 | 0.1 | 0.1 | 0.1 |

Reference example was prepared in accordance to WO 2008/024045.

### Process for preparation of pharmaceutical compositions of Examples 1-5

All examples stated above were prepared using wet granulation technology. Ticagrelor was homogeneously mixed with microcrystalline cellulose and calcium hydrogen phosphate, HPMC and sodium crosscarmellose. The mixture was granulated with water and dried to obtain dry granulate. Magnesium stearate was added and mixed. The resulted blend was compressed into tablets. In the last step film coating was applied by the processes known in the art.

### Process for preparation of Reference Example

Ticagrelor was homogeneously mixed with manitol and calcium hydrogen phosphate, HPC and sodium starch glycolate. The mixture was granulated with water and dried to obtain dry granulate. Magnesium stearate was added and mixed. The resulted blend was compressed into tablets. In the last step film coating was applied by the processes known in the art.

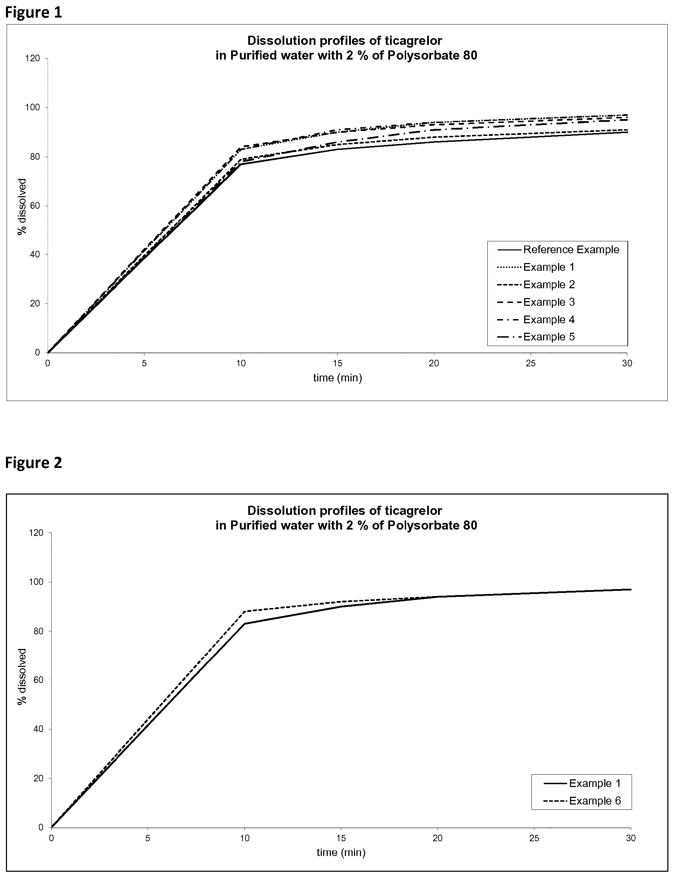

In the prior art it is disclosed that particle size of ticagrelor has high influence on dissolution profile and bioavailability of ticagrelor incorporated in pharmaceutical composition. As shown in Figure 1, in the formulation of the present invention a broad range of particle size of ticagrelor can be used for providing desired dissolution profile. Therefore, the formulation is not sensible in terms of variations of the active pharmaceutical ingredient, thereby providing for more robust technological process.

### Comparative Example 1

Robustness of the formulation of the present invention and formulation of the Reference Example comprising water soluble diluent/filler equivalent to the product on the market (Brilique) was tested. Reference Example was prepared according to the composition and process disclosed in WO 2008/024045.

Two reference examples with minimum and maximum amount of granulation liquid added and two examples according to the invention with minimum and maximum granulation liquid added were prepared. All formulations were prepared by the same procedure, where only the amount of granulation liquid in relation to the amount of granulation mixture was varied. Obtained ranges are presented in Table 2. Criterion for the minimum amount of water was the formation of granulate and the maximum amount was the point where the granulate was on the breaking point of still being processible, meaning that the granulate was maximally wetted and adding any additional water would lead to sticking of the granulate, it being unable to dry and could not be further used in the technological process. The results presented in Table 2 show that the robustness of formulation of the present invention is higher in comparison to the formulation comprising water soluble filler. Significantly higher range of applied granulation liquid and higher upper limit enable superior process control on industrial scale, whereby robust process control with small volumes of granulation liquid in narrow range is difficult to achieve.

**Table 2**

| | Range of granulation liquid to obtain processible granulate (w/w %) |
|---|---|
| Reference example | 15-35 |
| Example according to invention | 35-65 |

Weight percent (w/w %) are obtained by dividing the mass of the granulation liquid by the mass of granulation mixture prior to granulation.

### Example 6 (Reference example)

Pharmaceutical composition of Example 6 is presented in Table 3. In the example, a formulation comprising only one water insoluble diluent was prepared. The manufacturing procedure is similar to the procedure presented above for Examples 1-5.

**Table 3**

| | Example 6 [mg/tablet] |
|---|---|
| Ticagrelor | 90 |
| Microcrystalline cellulose MCC 101 | 189 |
| Hydroxypropylmethylcellulose 6CP/PHARMACOAT 606 | 9 |
| Sodium croscarmellose | 9 |
| Magnesium stearate | 3 |
| Coating | |
| Coating mixture | 8.9 |
| Ferrous oxide yellow | 0.1 |

According to prior art teaching at least one water soluble diluent must be used in the pharmaceutical compositions comprising ticagrelor. It has been found out by the present inventors that a pharmaceutical composition comprising water insoluble diluent can be used which is free of water-soluble diluent exhibiting improved dissolution profile. Examples 1-5 and 6 show that either combination of water-insoluble diluents or only one water insoluble diluent can be used. Dissolution profiles obtained are comparable (see Figure 2).

### Example 7

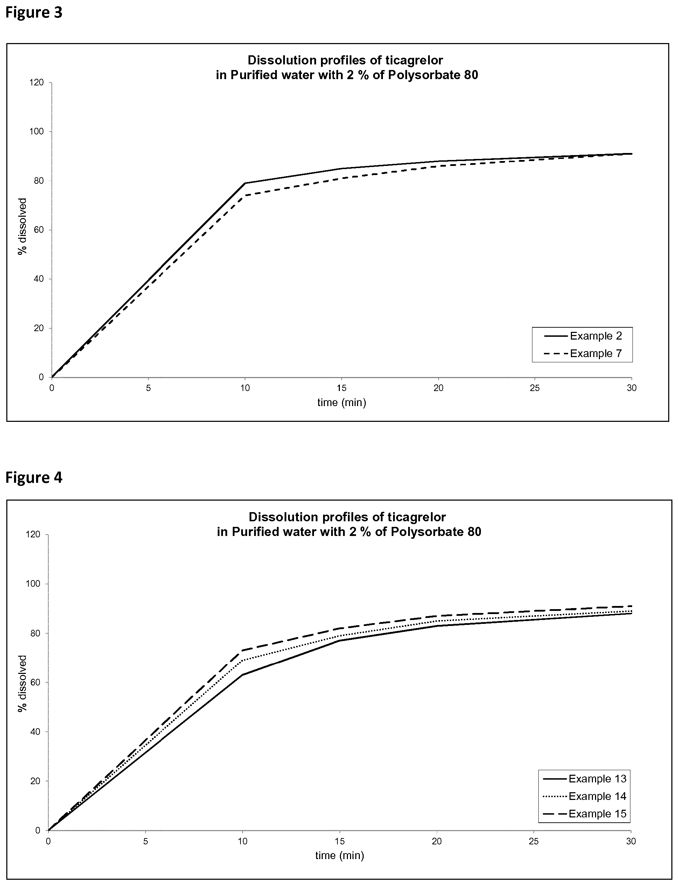

A pharmaceutical composition comprising 60 mg of ticagrelor is presented in Table 4. As shown in Figure 3 target processability, storage stability and a dissolution profile similar to that obtained with a formulation comprising 90 mg of ticagrelor is achieved.

**Table 4**

| | Example 7 [mg/tablet] |
|---|---|
| Ticagrelor | 60 |
| Microcrystalline cellulose MCC 101 | 84 |
| Calcium hydrogen phosphate | 42 |
| Hydroxypropylmethylcellulose 6CP/PHARMACOAT 606 | 6 |
| Sodium croscarmellose | 6 |
| Magnesium stearate | 2 |
| Coating | |
| Coating mixture | 5.989 |
| Ferrous oxide black | 0.001 |
| Ferrous oxide red | 0.01 |

Ticagrelor was homogeneously mixed with microcrystalline cellulose, calcium hydrogen phosphate, HPMC and sodium crosscarmellose. The mixture was granulated with water and dried to obtain a dry granulate. Magnesium stearate was added and mixed. The resulted blend was compressed into tablets. In the last step film coating was applied by the processes known in the art.

### Examples 8-12

| | **Example 8** | **Example 9** | **Example 10** | **Example 11** | **Example 12** |
|---|---|---|---|---|---|
| | **[mg/tbl]** | | | | |
| Ticagrelor (particle size approx. 9 µm) | 90 | 90 | 90 | 90 | 90 |
| Microcrystalline cellulose, type 101 | 53 | 70 | 70 | 53 | 128 |
| Calcium hydrogen phosphate | 128 | 113 | 114 | 128 | 53 |
| Hydroxypropyl methylcellulose 6CP/PHARMACOAT 606 | 6 | 9 | 10 | 9 | 6 |
| Sodium croscarmellose | 17 | 15 | 10 | 17 | 17 |
| Magnesium stearate | 6 | 3 | 6 | 3 | 6 |
| Coating | | | | | |
| Coating mixture | 8.9 | 8.9 | 8.9 | 8.9 | 8.9 |
| Ferrous oxide yellow | 0.1 | 0.1 | 0.1 | 0.1 | 0.1 |

### Process for preparation of pharmaceutical compositions of Examples 8-12

All examples stated above were prepared using wet granulation technology. Ticagrelor was homogeneously mixed with microcrystalline cellulose and calcium hydrogen phosphate, HPMC and sodium crosscarmellose. The mixture was granulated with water and dried to obtain dry granulate. Magnesium stearate was added and mixed. The resulted blend was compressed into tablets. In the last step film coating was applied by the processes known in the art.

### Examples 13-15

| | **Example 13** | **Example 14** | **Example 15** |
|---|---|---|---|
| | **[mg/tbl]** | | |
| Ticagrelor (particle size approx. 9 µm) | 90 | 90 | 90 |
| Microcrystalline cellulose, type 101 | 126 | 126 | 126 |
| Calcium hydrogen phosphate | 63 | 63 | 63 |
| Hydroxypropylmethylcellulose 6CP/PHARMACOAT 606 | 9 | 6.75 | 4.5 |
| Hydroxypropylmethylcellulose 6CP/PHARMACOAT 606 (dissolved in granulating liquid) | / | 2.25 | 4.5 |
| Sodium croscarmellose | 9 | 9 | 9 |
| Magnesium stearate | 3 | 3 | 3 |
| Coating | | | |
| Coating mixture | 8.9 | 8.9 | 8.9 |
| Ferrous oxide yellow | 0.1 | 0.1 | 0.1 |

### Process for preparation of pharmaceutical compositions of Examples 13-15

All examples stated above were prepared using wet granulation technology. Ticagrelor was homogeneously mixed with microcrystalline cellulose and calcium hydrogen phosphate, HPMC (in examples 14 and 15 only part of) and sodium crosscarmellose. The mixture was granulated with water (Example 13) or HPMC dissolved in water (Examples 14 and 15) and dried to obtain a dry granulate. Magnesium stearate was added and mixed. The resulted blend was compressed into tablets. In the last step film coating was applied by the processes known in the art. Using wet granulation technology with HPMC being partially dissolved in water (Examples 14 and 15) improved the obtained dissolution profiles (see Figure 4).

## Claims

1. A pharmaceutical formulation comprising (a) ticagrelor and (b) a mixture of at least two water insoluble fillers, wherein the formulation is free of water soluble fillers, and wherein the water insoluble fillers require more than 30 mL of water at a temperature of 25°C to dissolve 1 g of filler and wherein water soluble fillers require less than 30 mL of water at a temperature of 25°C to dissolve 1 g of filler.

2. The pharmaceutical formulation according to claim 1, comprising (a), as the active ingredient, ticagrelor, (b) a mixture of at least two water insoluble fillers, (c) at least one binder, (d) at least one disintegrant and (e) optionally further pharmaceutically acceptable excipients, wherein the formulation is free of water soluble fillers.

3. The pharmaceutical formulation according to claim 2, comprising (a), as the active ingredient, ticagrelor, (b) a mixture of at least two water insoluble fillers selected from polysaccharides, calcium salts of phosphates, sulphates and carbonates, (c) at least one binder selected from hydrophilic cellulose derivatives, (d) at least one disintegrant and (e) optionally further pharmaceutically acceptable excipients, wherein the formulation is free of water soluble fillers.

4. The pharmaceutical formulation according to claim 3, comprising (a), as the active ingredient, ticagrelor, (b) a mixture of at least two water insoluble fillers selected from microcrystalline cellulose, calcium hydrogen phosphate, calcium sulphate and calcium carbonate, (c) at least one binder selected from hydrophilic cellulose derivative, (d) at least one disintegrant and (e) optionally further pharmaceutically acceptable excipients, wherein the formulation is free of water soluble fillers.

5. The pharmaceutical formulation according to claim 1, wherein the at least two insoluble fillers (b) are selected from the group consisting of water insoluble polysaccharides, like cellulose, and calcium, sodium or potassium salts of phosphoric acid, carbonic acid or sulfuric acid and mixtures thereof.

6. The pharmaceutical formulation according to claim 5, wherein the at least two insoluble fillers (b) are a mixture of at least two water insoluble fillers selected from the group consisting of water insoluble polysaccharides and calcium salts of phosphoric acid, carbonic acid or sulfuric acid, and preferably are a mixture of microcrystalline cellulose and calcium hydrogen phosphate.

7. The pharmaceutical formulation according to claim 6, comprising (a), as the active ingredient, ticagrelor, (b) a mixture of at least two water insoluble fillers selected from microcrystalline cellulose and calcium hydrogen phosphate, (c) at least one binder selected from hydroxypropylmethylcellulose, (d) at least one disintegrant selected from sodium croscarmellose and (e) optionally further pharmaceutically acceptable excipients, wherein the formulation is free of water soluble fillers.

8. A pharmaceutical formulation according to claim 7, comprising 20 % to 50 % by weight of ticagrelor, 40 to 80 % by weight of a diluent selected from microcrystalline cellulose and calcium hydrogen phosphate, 1.5 to 10 % by weight of a binder selected from low viscosity hydroxypropylmethylcellulose and 1.5 to 10 % by weight of a disintegrant, preferably selected from croscarmellose sodium, and 0.5 to 3 % by weight of a lubricant, preferably selected from magnesium stearate.

9. The pharmaceutical formulation according to any one of claims 1 to 8, wherein the ticagrelor has a crystalline form.

10. The pharmaceutical formulation according to any one of claims 1 to 9, wherein the ticagrelor has a particle size distribution of d(0.1) of 0.1 to 20 µm, d(0.5) of 1 to 30 µm and d(0.9) of 3 to 100 µm.

11. A pharmaceutical formulation comprising (a) ticagrelor and (b) at least two water insoluble fillers, wherein the formulation comprises less than 5 % by weight, preferably less than 2 % by weight, more preferably less than 1 % by weight, of a water soluble filler, and wherein the water insoluble fillers require more than 30 mL of water at a temperature of 25°C to dissolve 1 g of filler and wherein the water soluble filler requires less than 30 mL of water at a temperature of 25°C to dissolve 1 g of filler.

12. A process for preparing the pharmaceutical formulation according to any one of claims 1-11 by wet granulation, which process comprises:
- blending optionally sieved ticagrelor with at least one excipient or a mixture of excipients to obtain a granulation mixture;
- granulating the granulation mixture with a granulation liquid using processes known in the art, such as fluid bed granulator or high shear granulator, to obtain a granulate;
- optionally drying, milling and sieving the obtained granulate to obtain a granulate with the desired particle size distribution;
- adding the rest of the at least one excipient or a mixture of excipients to the granulate to give a mixture; and
- compressing the mixture to the desired solid dosage form or encapsulating the mixture.

13. A process for preparing the pharmaceutical formulation according to any one of claims 1-11, which comprises the following steps:
- blending ticagrelor and at least one excipient or a mixture of excipients, which excipient(s) is/are optionally sieved, to give a granulation mixture;
- granulating the granulation mixture using a granulation liquid which comprises water and optionally binder to give a granulate;
- optionally drying, milling and sieving the obtained granulate;
- adding lubricant to the granulate to give a compression mixture;
- compressing the compression mixture to the desired solid dosage form or encapsulating the compression mixture;
- optionally, applying a film coating.

14. A process according to claim 12 or 13, wherein the granulation liquid comprises water and optionally at least a part of the binders present in the pharmaceutical composition, wherein preferably the granulation liquid comprises water and a part of the total amount of binders present in the pharmaceutical composition, such as one quarter or one half of the total amount of binders present in the formulation.

## Patentansprüche

1. Pharmazeutische Formulierung, die (a) Ticagrelor und (b) eine Mischung aus mindestens zwei wasserunlöslichen Füllstoffen enthält, wobei die Formulierung frei von wasserlöslichen Füllstoffen ist und wobei die wasserunlöslichen Füllstoffe bei einer Temperatur von 25°C mehr als 30 ml Wasser benötigen, um 1 g Füllstoff zu lösen, und wobei wasserlösliche Füllstoffe bei einer Temperatur von 25°C weniger als 30 ml Wasser benötigen, um 1 g Füllstoff zu lösen.

2. Pharmazeutische Formulierung nach Anspruch 1, die (a) Ticagrelor als Wirkstoff, (b) eine Mischung aus mindestens zwei wasserunlöslichen Füllstoffen, (c) mindestens ein Bindemittel, (d) mindestens ein Sprengmittel und (e) gegebenenfalls weitere pharmazeutisch annehmbare Hilfsstoffe enthält, wobei die Formulierung frei von wasserlöslichen Füllstoffen ist.

3. Pharmazeutische Formulierung nach Anspruch 2, die (a) Ticagrelor als Wirkstoff, (b) eine Mischung aus mindestens zwei wasserunlöslichen Füllstoffen ausgewählt aus Polysacchariden, Calciumsalzen von Phosphaten, Sulfaten und Carbonaten, (c) mindestens ein Bindemittel ausgewählt aus hydrophilen Cellulosederivaten, (d) mindestens ein Sprengmittel und (e) gegebenenfalls weitere pharmazeutisch annehmbare Hilfsstoffe enthält, wobei die Formulierung frei von wasserlöslichen Füllstoffen ist.

4. Pharmazeutische Formulierung nach Anspruch 3, die (a) Ticagrelor als Wirkstoff, (b) eine Mischung aus mindestens zwei wasserunlöslichen Füllstoffen ausgewählt aus mikrokristalliner Cellulose, Calciumhydrogenphosphat, Calciumsulfat und Calciumcarbonat, (c) mindestens ein Bindemittel ausgewählt aus einem hydrophilen Cellulosederivat, (d) mindestens ein Sprengmittel und (e) gegebenenfalls weitere pharmazeutisch annehmbare Hilfsstoffe enthält, wobei die Formulierung frei von wasserlöslichen Füllstoffen ist.

5. Pharmazeutische Formulierung nach Anspruch 1, wobei die mindestens zwei unlöslichen Füllstoffe (b) aus der Gruppe ausgewählt sind, die aus wasserunlöslichen Polysacchariden, wie Cellulose, und Calcium-, Natrium- oder Kaliumsalzen von Phosphorsäure, Kohlensäure oder Schwefelsäure und Mischungen davon besteht.

6. Pharmazeutische Formulierung nach Anspruch 5, wobei die mindestens zwei unlöslichen Füllstoffe (b) eine Mischung aus mindestens zwei wasserunlöslichen Füllstoffen, ausgewählt aus Gruppe bestehend aus wasserunlöslichen Polysacchariden und Calciumsalzen von Phosphorsäure, Kohlensäure oder Schwefelsäure, und vorzugsweise eine Mischung aus mikrokristalliner Cellulose und Calciumhydrogenphosphat sind.

7. Pharmazeutische Formulierung nach Anspruch 6, die (a) Ticagrelor als Wirkstoff, (b) eine Mischung aus mindestens zwei wasserunlöslichen Füllstoffen ausgewählt aus mikrokristalliner Cellulose und Calciumhydrogenphosphat, (c) mindestens ein Bindemittel ausgewählt aus Hydroxypropylmethylcellulose, (d) mindestens ein Sprengmittel ausgewählt aus Natriumcroscarmellose, und (e) gegebenenfalls weitere pharmazeutisch annehmbare Hilfsstoffe enthält, wobei die Formulierung frei von wasserlöslichen Füllstoffen ist.

8. Pharmazeutische Formulierung nach Anspruch 7, die 20 bis 50 Gew.-% Ticagrelor, 40 bis 80 Gew.-% eines Verdünnungsmittels ausgewählt aus mikrokristalliner Cellulose und Calciumhydrogenphosphat, 1,5 bis 10 Gew.-% eines Bindemittels ausgewählt aus niedrigviskoser Hydroxypropylmethylcellulose und 1,5 bis 10 Gew.-% eines Sprengmittels, vorzugsweise ausgewählt aus Croscarmellose-Natrium, und 0,5 bis 3 Gew.-% eines Gleitmittels, vorzugsweise ausgewählt aus Magnesiumstearat, enthält.

9. Pharmazeutische Formulierung nach einem der Ansprüche 1 bis 8, wobei das Ticagrelor eine kristalline Form aufweist.

10. Pharmazeutische Formulierung nach einem der Ansprüche 1 bis 9, wobei das Ticagrelor eine Teilchengrößenverteilung von d(0,1) von 0,1 bis 20 µm, d(0,5) von 1 bis 30 µm und d(0,9) von 3 bis 100 µm aufweist.

11. Pharmazeutische Formulierung, die (a) Ticagrelor und (b) mindestens zwei wasserunlösliche Füllstoffe enthält, wobei die Formulierung weniger als 5 Gew.- %, vorzugsweise weniger als 2 Gew.-%, noch bevorzugter weniger als 1 Gew.-%, eines wasserlöslichen Füllstoffs umfasst und wobei die wasserunlöslichen Füllstoffe bei einer Temperatur von 25°C mehr als 30 ml Wasser benötigen, um 1 g Füllstoff zu lösen, und wobei der wasserlösliche Füllstoff bei einer Temperatur von 25°C weniger als 30 ml Wasser benötigt, um 1 Füllstoff zu lösen.

12. Verfahren zur Herstellung der pharmazeutischen Formulierung nach einem der Ansprüche 1-11 durch Nassgranulation, wobei bei dem Verfahren:
- gegebenenfalls gesiebtes Ticagrelor mit mindestens einem Hilfsstoff oder einer Mischung von Hilfsstoffen vermischt wird, um eine Granulatmischung zu erhalten;
- die Granulatmischung mit einer Granulierflüssigkeit unter Verwendung von in der Technik bekannten Verfahren, wie z.B. Wirbelschichtgranulator oder Hochschergranulator, granuliert wird, um ein Granulat zu erhalten;
- das erhaltene Granulat gegebenenfalls getrocknet, gemahlen und gesiebt wird, um ein Granulat mit der gewünschten Teilchegrößenverteilung zu erhalten;
- der restliche mindestens eine Hilfsstoff oder eine Mischung von Hilfsstoffen zu dem Granulat gegeben wird, um eine Mischung zu erhalten; und
- die Mischung zur gewünschten festen Darreichungsform verpresst wird oder die Mischung in Kapselform überführt wird.

13. Verfahren zur Herstellung der pharmazeutischen Formulierung nach einem der Ansprüche 1-11, bei dem:
- Ticagrelor und mindestens ein Hilfsstoff oder eine Mischung von Hilfsstoffen vermischt werden, wobei der/die Hilfsstoff(e) gegebenenfalls gesiebt wird/werden, um eine Granulationsmischung zu erhalten;
- die Granulatmischung mit einer Granulierflüssigkeit, die Wasser und gegebenenfalls Bindemittel enthält, granuliert wird, um ein Granulat zu erhalten;
- das erhaltene Granulat gegebenenfalls getrocknet, gemahlen und gesiebt wird;
- Gleitmittel zum Granulat gegeben wird, um eine Kompressionsmischung zu erhalten;
- die Kompressionsmischung zur gewünschten festen Darreichungsform verdichtet wird oder die Kompressionsmischung in Kapselform überführt wird;
- gegebenenfalls eine Filmbeschichtung aufgebracht wird.

14. Verfahren nach Anspruch 12 oder 13, wobei die Granulierflüssigkeit Wasser und gegebenenfalls mindestens einen Teil der in der pharmazeutischen Zusammensetzung vorhandenen Bindemittel umfasst, wobei die Granulierflüssigkeit vorzugsweise Wasser und einen Teil Gesamtmenge der in der pharmazeutischen Zusammensetzung vorhandenen Bindemittel umfasst, wie beispielsweise ein Viertel oder die Hälfte der Gesamtmenge der in der Formulierung vorhandenen Bindemittel.

## Revendications

1. Formulation pharmaceutique comprenant (a) du ticagrelor et (b) un mélange d'au moins deux charges insolubles dans l'eau, dans laquelle la formulation est exempte de charges solubles dans l'eau, et dans laquelle les charges insolubles dans l'eau nécessitent plus de 30 ml d'eau à une température de 25°C pour dissoudre 1 g de charge et dans laquelle les charges solubles dans l'eau nécessitent moins de 30 ml d'eau à une température de 25°C pour dissoudre 1 g de charge.

2. Formulation pharmaceutique selon la revendication 1, comprenant (a), en tant que principe actif, du ticagrelor, (b) un mélange d'au moins deux charges insolubles dans l'eau, (c) au moins un agent de liaison, (d) au moins un agent de désintégration et (e) éventuellement d'autres excipients pharmaceutiquement acceptables, dans laquelle la formulation est exempte de charges solubles dans l'eau.

3. Formulation pharmaceutique selon la revendication 2, comprenant (a) en tant que principe actif, du ticagrelor, (b) un mélange d'au moins deux charges insolubles dans l'eau choisies parmi des polysaccharides, des sels de type calcium de phosphate, de sulfate et de carbonate, (c) au moins un agent de liaison choisi parmi des dérivés de cellulose hydrophile, (d) au moins un agent de désintégration et (e) éventuellement d'autres excipients pharmaceutiquement acceptables, dans laquelle la formulation est exempte de charges solubles dans l'eau.

4. Formulation pharmaceutique selon la revendication 3 comprenant (a), en tant que principe actif, du ticagrelor, (b) un mélange d'au moins deux charges insolubles dans l'eau choisies parmi la cellulose microcristalline, l'hydrogénophosphate de calcium, le sulfate de calcium et le carbonate de calcium, (c) au moins un agent de liaison choisi parmi un dérivé de cellulose hydrophile, (d) au moins un agent de désintégration et (e) éventuellement d'autres excipients pharmaceutiquement acceptables, dans laquelle la formulation est exempte de charges solubles dans l'eau.

5. Formulation pharmaceutique selon la revendication 1, dans laquelle les charges insolubles au nombre d'au moins deux (b) sont choisies dans le groupe comprenant des polysaccharides insolubles dans l'eau, tels que la cellulose, et des sels de calcium, de sodium ou de potassium d'acide phosphorique, d'acide carbonique ou d'acide sulfurique et leurs mélanges.

6. Formulation pharmaceutique selon la revendication 5, dans laquelle les charges insolubles au nombre d'au moins deux (b) sont un mélange d'au moins deux charges insolubles dans l'eau choisies dans le groupe comprenant des polysaccharides insolubles dans l'eau et des sels de calcium d'acide phosphorique, d'acide carbonique ou d'acide sulfurique, et sont de préférence un mélange de cellulose microcristalline et d'hydrogénophosphate de calcium.

7. Formulation pharmaceutique selon la revendication 6, comprenant (a) en tant que principe actif, du ticagrelor, (b) un mélange d'au moins deux charges insolubles dans l'eau choisies parmi la cellulose microcristalline et l'hydrogénophosphate de calcium, (c) au moins un agent de liaison choisi parmi l'hydroxypropylméthylcellulose, (d) au moins un agent de désintégration choisi parmi la croscarmellose sodique et (e) éventuellement d'autres excipients pharmaceutiquement acceptables, dans laquelle la formulation est exempte de charges solubles dans l'eau.

8. Formulation pharmaceutique selon la revendication 7, comprenant de 20 % à 50 % en poids de ticagrelor, de 40 % à 80 % en poids d'un diluant choisi parmi la cellulose microcristalline et l'hydrogénophosphate de calcium, de 1,5 à 10 % en poids d'un agent de liaison choisi parmi l'hydroxypropylméthylcellulose à faible viscosité et de 1,5 à 10 % en poids d'un agent de désintégration, de préférence choisi parmi la croscarmellose sodique, et de 0,5 à 3 % en poids d'un lubrifiant, de préférence choisi parmi le stéarate de magnésium.

9. Formulation pharmaceutique selon l'une quelconque des revendications 1 à 8, dans laquelle le ticagrelor est sous forme cristalline.

10. Formulation pharmaceutique selon l'une quelconque des revendications 1 à 9, dans laquelle le ticagrelor présente une distribution granulométrique d(0.1) de 0,1 à 20 µm, d(0.5) de 1 à 30 µm et d(0.9) de 3 à 100 µm.

11. Formulation pharmaceutique comprenant (a) du ticagrelor et (b) au moins deux charges insolubles dans l'eau, dans laquelle la formulation comprend moins de 5 % en poids, de préférence moins de 2 % en poids, de préférence moins de 1 % en poids, d'une charge soluble dans l'eau et dans laquelle les charges insolubles dans l'eau nécessitent plus de 30 ml d'eau à une température de 25°C pour dissoudre 1 g de charge et dans laquelle la charge soluble dans l'eau nécessite moins de 30 ml d'eau à une température de 25°C pour dissoudre 1 g de charge.

12. Procédé de préparation de la formulation pharmaceutique selon l'une quelconque des revendications 1 à 11 par granulation humide, comprenant le fait de :
- mélanger éventuellement du ticagrelor tamisé à au moins un excipient ou un mélange d'excipients afin d'obtenir un mélange de granulation ;
- granuler le mélange de granulation à l'aide d'un liquide de granulation à l'aide de procédés connus dans l'art, tels qu'un granulateur en lit fluidisé ou un granulateur à haut cisaillement, afin d'obtenir un granulé ;
- éventuellement, sécher, broyer et tamiser le granulé obtenu afin d'obtenir un granulé présentant la distribution granulométrique souhaitée ;
- ajouter le reste d'au moins un excipient ou un mélange d'excipients au granulé afin d'obtenir un mélange ; et
- comprimer le mélange pour obtenir la forme posologique solide souhaitée ou encapsuler le mélange.

13. Procédé de préparation de la formulation pharmaceutique selon l'une quelconque des revendications 1 à 11, qui comprend les étapes suivantes consistant à :
- mélanger du ticagrelor et au moins un excipient ou un mélange d'excipients, le(s)quel(s) excipient(s) est(sont) éventuellement tamisé(s), afin d'obtenir un mélange à granuler ;
- granuler le mélange à granuler à l'aide d'un liquide de granulation qui comprend de l'eau et éventuellement un agent de liaison afin d'obtenir un granulé ;
- éventuellement, sécher, broyer et tamiser le granulé obtenu ;
- ajouter un lubrifiant au granulé afin d'obtenir un mélange à comprimer ;
- comprimer le mélange à comprimer pour obtenir la forme posologique solide souhaitée ou encapsuler le mélange à comprimer ;
- éventuellement, appliquer un enrobage.

14. Procédé selon la revendication 12 ou la revendication 13, dans lequel le liquide de granulation comprend de l'eau et éventuellement au moins une partie des agents de liaison présents dans la composition pharmaceutique, dans lequel le liquide de granulation comprend de préférence de l'eau et une partie de la quantité totale d'agent de liaison présent dans la composition pharmaceutique, de telle sorte qu'un quart ou la moitié de la quantité totale d'agent de liaison est présent dans la formulation.
